# EUROPEAN PATENT SPECIFICATION

(11) **EP 1 468 675 B1**
(45) Date of publication and mention of the grant of the patent: **21.01.2009**
(21) Application number: 04252191.4
(22) Date of filing: 14.04.2004
(51) Int. Cl.: A61K 8/04, A61K 8/39, A61Q 19/10

(54) **Post-foaming cleansing composition**
Nachschäumendes Reinigungsmittel
Composition moussante de nettoyage

(30) Priority: 14.04.2003 GB 0308585
(43) Date of publication of application: 20.10.2004
(73) Proprietor: PZ Cussons (International) Limited, Stockport SK3 0XN (GB)
(72) Inventor: Yaqub, Najem, Oldham Lancs, OL( 4AG (GB); Bullen, Roxanne, Didsbury Manchester, M20 6WB (GB); Atkinson, Helen Patricia, Gatley Cheadle, SK8 4HP (GB)
(74) Representative: Wilson Gunn

(56) References cited:
- EP-A- 0 437 956
- WO-A-97/03646
- WO-A-97/20626
- WO-A-99/32070
- GB-A- 2 213 160
- US-A1- 2001 013 352
- US-B1- 6 210 656

## Description

The present invention relates to a cleansing composition and more particularly a personal cleansing composition intended for use in a shower.

Personal cleansing compositions have been developed for use in showers, such as shower gels. However, large numbers of people prefer to use a conventional bar of soap rather than a shower gel. It is believed that one factor responsible for resistance to the use of shower gels is connected to lather generation. In order to produce lather from a shower gel the user must apply shear to the gel. In some cases much of this effort is wasted as the lather washes away before it can be applied to the body.

In order to deal with the problem it is known to provide a shower gel which creates lather as soon as possible after the gel is dispensed from the package.

WO 96/09032A discloses a soap-free post-foaming gel composition which is particularly intended for shaving using a razor. The composition utilises a volatile hydrocarbon such as isopentane to provide a gel structure. Although this composition is satisfactory for its intended purpose it does not perform well for personal washing mainly because it gives an uncomfortable 'stripped' feeling to the skin.

WO 97/03646 discloses a post-foaming gel composition for use in an aerosol container wherein the composition comprises a base material having a viscosity of at least 9500 cps to which is added a foam-forming propellant gas. Whilst this composition has good personal washing properties its viscous nature makes it particularly difficult to process and package and thus there is a high degree of wastage during manufacture.

Attempts have been made to address this problem. For example, WO00/39273 discloses a post-foaming composition of low viscosity which gels upon addition of the post-foaming agent. However, this sudden, instantaneous gelling can make the composition difficult to fill into suitable packaging and may lead to stoppages and breakdowns in the pipe-work of the plant machinery resulting in plant downtime and inefficient production. This formulation must be filled into required packaging immediately on addition of the post-foaming agent.

Similarly, GB 2,213,160 teaches of a post foaming gel product containing an anionic surfactant and a non-ionic, ethoxylated fatty alcohol or ester. The product gels on addition of post-foaming agent and forms a viscous gel prior to filling into a suitable container. This again presents difficulties in terms of manufacturing due to the high viscosity of the gel formed.

WO 02/05758 discloses a self foaming composition wherein the composition is in the form of a liquid crystalline structure. This results in a soft gel of lower viscosity. Whilst the soft gel is easier to spread onto the skin it has a number of disadvantages compared to a more structured viscous gel. A more viscous gel will release the post-foaming agent more slowly on agitation and lather is developed in a more controlled manner. Thus, it appears that the user is responsible for creating the foam as with conventional cleaning materials and the composition is perceived as providing the behaviour expected for a good cleaning operation. The lather generated from a more viscous gel is creamier than that generated from a soft gel. An additional benefit of a viscous gel is that the product can be spread further over the body without being washed away as with conventional products. Thus it appears to the user that the product is more economical.

Many of the post-foaming compositions currently available have an opaque or cloudy appearance. This provides poor visual differentiation between the initial gel which is dispensed and the lather generated when the gel is spread onto the skin, as such these compositions lack visual appeal.

A common problem with currently available post-foaming compositions is their poor long term stability. Compositions having poor long term stability have a limited shelf life. Clearly, this restricts the commercial viability of such compositions as poor long term stability can lead to product wastage should the product not sell etc.

It is desirable therefore to provide a post-foaming cleansing composition which is sufficiently mobile to facilitate easy processing and packaging, but which has a stable, clear, viscous gel structure when dispensed from its packaging which enables slow release of a post-foaming agent and provides a creamy lather and moreover a viscous gel structure that is maintained throughout a reasonable product lifetime, under ambient conditions i.e. 20 to 25°C. It is also desirable to provide a post-foaming cleansing composition which gives acceptable post washing skin feel i.e. does not the leave the skin feeling dry and tight.

According to the present invention there is provided a method for the manufacture of a post-foaming cleansing composition comprising the steps of:- adding at least one non-ionic gelling agent to a mixture comprising at least one anionic surfactant such that the ratio of anionic surfactants to non-ionic gelling agent is 4:1 or greater, the method further comprising combining the ensuing mixture with at least one post-foaming agent and filling the mixture into a package prior to the formation of a gel structure, and wherein a gel structure is formed at least 4 minutes after the addition of the post-foaming agent to the mixture, said gel structure being retained for at least 12 months following manufacture when the composition is stored at about 20°C to 25°C or below.

Advantageously, due to the presence of the non-ionic gelling agent, once the composition has been filled into the packaging and allowed to stand the viscosity of the composition and therefore its gel rigidity increases. This gives rise to a composition which provides a non-mobile, shear thinning viscous gel which remains substantially unchanged until dispensed from the packaging for at least 12 months following manufacture when the composition is stored at 20°C to 25°C.

A further advantage of the invention is that the gel structure of the composition of the present invention does not begin to form for a sufficient period of time during the processing and packaging of the composition such that it can be easily pumped and filled into the packaging. Furthermore, there is no gel formation within plant pipe-work and so stoppages and breakages are minimised.

A further advantage of the present invention is that due to the delayed gelling elevated pressure is not required to pump the composition through the pipe-work. This not only reduces the manufacturing costs of the end product, but it also increases the filling rates meaning more units of composition of the present invention can be produced in the same time period relative to previously available compositions.

A still further advantage of the present invention is that the long term stability of the product is improved i.e. the gel structure and thus its performance properties remain consistent throughout the anticipated shelf-life of the product.

Thus, neither the appearance of the gel on first dispensing from the packaging nor the quality of the lather produced upon agitating the gel by the user is compromised for the sake of ease of manufacturing. Furthermore, the physical attributes of the gel are maintained throughout the lifetime of the product.

A further advantage of the present invention is that the gels produced are clear. This clarity of the gel provides an indication to the consumer of the high product quality and further differentiates visually the product when initially dispensed from the foam produced on spreading the product. A clear gel also makes the inclusion of pigments much easier. Furthermore, the clarity of the gel of the present invention remains substantially unchanged throughout the lifetime of the product.

Due to the volatility of the post-foaming agent, it is not possible to accurately measure the viscosity of the composition following the addition of the post-foaming agent i.e. the gelled composition. Therefore, the gel rigidity of the composition is used as a measure of the extent to which a composition has gelled.

The gel rigidity test is conducted at ambient temperature of 20 to 25°C where both the can and its contents are at this temperature. The can is held approximately 2 inches above a white sheet of paper and actuated for 2 seconds to dispense the gel. The characteristics of the gel are observed and scored for Rigidity (R ) using the following arbitrary rigidity rating scale:-
R = 1 Rigid gel with no visible mobility
Gel retains its shape until the post-foaming agent starts to be released and the gel starts to turn into a foam
R = 2 Firm gel with some visible mobility
Gel starts to form into an amorphous mound before the post-foaming agent starts to be released and the gel starts to turn into a foam
R = 3 Firm gel with some visible mobility which collapses slowly
   Gel forms an amorphous mound on dispensing which slowly spreads
R = 4 Thin gel/mobile liquid
   Gel has viscous appearance but spreads rapidly after dispensing
R = 5 Thin liquid
   Gel has a non-viscous appearance and is runny after dispensing

By substantially unchanged it is meant that the score for gel rigidity of the composition does not change by more than 1 unit on the rigidity rating scale.

A particularly important feature of the composition of the present invention is the non-ionic gelling agent. It is thought that it is the combination of the non-ionic gelling agent with the surfactants of the present invention which is responsible for the long term stability of the gel structure.

Preferably the non-ionic gelling agent is added in such a quantity that the final gel structure is rigid and scores a rating of R = 1 on the rigidity rating scale. If the quantity of non-ionic gelling agent is too small then a gel rigidity of R=1 is not achieved. Compositions with a gel rigidity of R=3 or softer have poor long term stability and do not maintain the gel structure throughout the lifetime of the product. Products with good long term stability maintain the structure of the gel such that on dispensing the gel, the gel rigidity score does not change by more than 1 unit on the rigidity rating scale throughout the lifetime of the product.

It is thought that the non-ionic gelling agent is also responsible for the delayed gelling of the composition following addition of the post foaming agent.

Upon addition of the post-foaming agent the composition has a gel rigidity rating score of 3 units or more and more preferably 4 units or more. At this time the composition can be described as a thin mobile gel or liquid. Once the composition begins to form a gel structure it does so such that the composition has a final gel rigidity rating score ofR= 1 or 2 or more preferably R= 1.

Preferably the gel rigidity of the composition remains substantially unchanged for at least 10 minutes after addition of the post-foaming agent to the remainder of the composition and most preferably for at least 30 minutes after addition of the post-foaming agent to the remainder of the composition.

It is difficult to measure clarity of a post foaming gel when first dispensed because it will start to become cloudy on release of the post foaming agent from the gel structure. For this reason the clarity of the gel is measured using a Gel Clarity Rating scale.

The gel clarity test is conducted at ambient temperature of 20°C to 25°C, where both the can and its contents are at this temperature. The can is held approximately 2 inches above a white sheet of feint ruled paper and actuated to dispense a 3 inch line of gel.

The characteristics of the gel are observed and scored for Clarity using the following arbitrary clarity rating scale:-

| | | |
|---|---|---|
| C = 1 | Perfect Clarity | Clear gel, Lines are easily defined |
| C = 2 | Standard Clarity | Clear gel, lines are visible |
| C = 3 | Minor Clouding | Hazy Gel ― Lines are not clearly defined |
| C = 4 | Major Clouding | Hazy Gel ― lines are not clearly visible |
| C = 5 | Critical Clouding | Cloudy gel ― lines are obscured |

It is desirable therefore that the formulation when dispensed forms a gel with a clarity rating score of C = 1 or 2, more preferably C = 1.

By substantially unchanged it is meant that the score for gel clarity of the composition does not change by more than 1 unit on the clarity rating scale.

Suitable non-ionic gelling agents which can be used alone or in combination include alkoxylated alcohols, glyceryl esters, glycol esters, alkoxylated carboxylic acids, alkanolamides and their derivatives. Preferred non-ionic gelling agents include alkoxylated alcohols such as laureth-2, laureth-4, C12/13 pareth-3, ceteareth-4 or oleth-3 or glycol esters such as coconut fatty acid monoglyceride polyglycol ether or modified palm oil polyglycol ether.

The particularly preferred non-ionic gelling agents are laureth-4 and/or PEG 7 Glyceryl cocoate.

The non-ionic gelling agent shall preferably constitute from 0.01% to 8.00% by weight of the total composition, more preferably form 0.01% to 4.0% by weight and most preferably from 0.5% to 2.5 % by weight of the total composition.

Suitable anionic surfactants include alkali metal alkyl ether sulfates, sulfosuccinates, isethionates and acyl glutamates. In addition to at least one anionic surfactant the composition of the present invention preferably further comprises at least one amphoteric surfactant such as betaines.

A particularly preferred anionic surfactant is sodium lauryl ether sulphate and particularly preferred amphoteric surfactant is cocamidopropyl betaine.

The total surfactant shall preferably constitute from 0.01% to 30.0% by weight of the total composition and more preferably from 15% to 28% by weight of the total composition and most preferably from 18% to 25% by weight of the total composition. Where the surfactant component comprises more than one surfactant the anionic surfactant shall preferably be the major surfactant and as such constitute at least 50% by weight of the total surfactant.

In addition to the at least one anionic surfactant the composition of the present invention may comprise further surfactants, which may be used alone or in combination, and include any of the following:- anionic, cationic, non-ionic, amphoteric (zwitter-ionic) surfactants.

Specific surfactants which may be used alone or in combination include any of the following:- alkyl polyglucosides, ethoxylated and non-ethoxylated metal alkyl sulfates, sultaines, taurates, betaines, sarcosinates, sulfosuccinates, sulfonates, carboxylates, glycinates, amphoacetates, amphodiacetates, isethionates, quaternary ammonium compounds, polysorbates, sugar esters, alkyl phosphates, propionates, amino acid surfactants, glucosides, acyl glutamates, alkanolamides and betaines.

The post-foaming agent used in the composition of the present invention is chosen largely out of consideration for the particular type of composition that is being formulated.

The preferred post-foaming agent comprises at least one saturated aliphatic hydrocarbon having from 4 to 6 carbons such as n-butane, iso-butane, n-pentane, isopentane, iso- hexane and mixtures thereof.

The post-foaming agent preferably constitutes from 0.01% to 14% by weight and most preferably from 7% to 11 % by weight of the total composition.

Additional ingredients may be added to the composition of the present invention including any of the following:- fragrances, essential oils, plant extracts, antimicrobial agents, colouring agents, skin conditioning agents, humectants preservatives, pearlisers, opacifiers, pH modifiers, shimmering agents, exfolliants, silicone oils, lipids, vitamins, sunscreen agents, skin lightening agents, and mixtures thereof.

Suitable additional ingredients include those which do not affect the gel rigidity of the compositions of the present invention thereby avoiding a low viscosity product.

Typically, at a least some of the ingredients of cleansing compositions such as those described herein, are solids and/or powders. This can give rise to mixing problems as it is more difficult to achieve a homogeneous mixture when some of the components are solids. Furthermore, it may be necessary to heat some of the components thus providing a hot batch mixing process. This introduces extra processing steps and manufacturing costs.

Thus, the components of the present invention are preferably liquid in order that manufacturing is facilitated.

The present invention is packaged in a container that is suitable for dispensing a post-foaming gel for example a bag on valve system, a bag in can system or an elasticated bladder container. These types of containers are well known to those skilled in the art.

In order that the present invention is more readily understood the composition of the present invention will now be described further by way of example only and with reference to the following examples.

### (Base formulation for examples 1a to 1d)

| **Component** | **Function** | **Quantity (% w/w)** |
|---|---|---|
| Water | Solvent | 68.80 - 75.00 |
| Disodium EDTA | Chelating Agent | 0.05 - 0.30 |
| Citric Acid | pH Adjuster | 0.05 - 0.30 |
| Methyldibromo Glutaronitrile and Phenoxyethanol | Preservative | 0.05 - 0.10 |
| Sodium Laureth Sulfate | Primary Surfactant | 17.50 - 22.00 |
| Cocamidopropyl Betaine | Foam Booster | 0.5 - 3.00 |
| PEG-7 Glyceryl Cocoate | Skin conditioning agent/non-ionic gelling agent | 0.50 - 1.50 |
| Glycerin | Skin conditioning agent | 0.10 - 2.00 |
| Colour | Colourant | 0.0005 - 0.0010 |
| Parfum | Masking Agent | 0.70 |
| Laureth-4 | Gelling Agent | As table below |

### Examples 1a - 1d

| **Formulation** | **% Laureth 4** | **Viscosity @ 20°C** | **Appearance of Gel Concentrate** | **Time to reach R=1** | **Finished Product Gel Appearance/Rigidiy** |
|---|---|---|---|---|---|
| 1a | 0 | 226cP (Spindle RV 4 Speed 10) | Clear Thin Liquid | Achieves R=2/3 after 60 minutes Does not achieve R=1 | Soft Cloudy Gel R=2/3 C=4 |
| 1β | 1.00 | 750cP (Spindle RV 4 Speed 10) | Clear Thin Liquid | 40 minutes | Clear Rigid Gel R = 1 C=1 |
| 1c | 1.25 | 1020cP (Spindle RV 4 Speed 10) | Clear Thin Liquid | 20 minutes | Clear Rigid Gel R=1 C=1 |
| 1d | 8.25 | 424,000cP (Spindle RV 5 Speed 0.5) | Cloudy Viscous Gel | Instantly achieves R=2/3 Does not achieve R=1 | Soft Cloudy Gel R=2/3 C=4 |

Viscosity is measured on a Brookfield Sinchro-lectric Viscometer
Time to reach R=1 (mins) refers to the time taken, in minutes, for the composition to form a rigid gel with no visible rigidity following addition of the post foaming agent. Viscosity is measured on a Brookfield viscometer at 20°C.

### Example 2

| **Component** | **Function** | **% w/w** | |
|---|---|---|---|
| | | **2a** | **2b** |
| Water | Solvent | qs | qs |
| Disodium EDTA | Chelating Agent | 0.1 | 0.1 |
| Citric Acid | pH Adjuster | 0.05 | 0.05 |
| Methyldibromo Glutaronitrile and Phenoxyethanol | Preservative | 0.08 | - |
| Sodium benzoate | | - | 1.0 |
| Sodium Laureth Sulfate | Primary Surfactant | 18.25 | 12.6 |
| Cocamidopropyl Betaine | Foam Booster | 1.33 | 4.2 |
| Capryl/capramidopropyl Betaine | Foam Booster | 0.35 | - |
| Guar Hydroxypropyltrimmonium Chloride | Skin conditioning agent | - | 0.1 |
| PEG-7 Glyceryl Cocoate | Skin conditioning agent/Non-ionic gelling agent | 1.00 | - |
| Isopropyl palmitate | | - | 1.5 |
| PEG 120 Methyl glucose dioleate | | - | 2.0 |
| PEG 40 Hydrogenated Castor Oil | | - | 0.5 |
| Glycerin | Skin conditioning agent | 0.1 | - |
| Colour | Colourant | 0.0005-0.0010 | 0.0005-0.0010 |
| Parfum | Masking Agent | 0.70 | 1.0 |
| Laureth-4 | Gelling Agent | 1.25 | 0 |

Compositions were prepared by the same method as example 1. The composition was filled into a suitable bag-in can aerosol container and tested for gel rigidity.
The samples were stored at the following conditions:
3°C
Ambient 20 to 25°C
37°C
45°C
Samples were removed from the storage conditions at monthly intervals:
At each interval the containers and their contents were equilibrated to ambient temperature of 20 to 25°C. The gel rigidity test was conducted as described herein. In addition the appearance of the gel was assessed.

| **Rigidity scores R = 1 -5** | | | | |
|---|---|---|---|---|
| **Example** | **Storage temperature** | **30 minutes** | **26 weeks** | **52 weeks** |
| 2a | 3°C | - | 1 | 1 |
| | 20-25°C | 1 | 1 | 1 |
| | 37°C | - | 3 | 3 |
| 2b | 3°C | - | 3 | 4 |
| | 20-25°C | 1 | 4 | 5 |
| | 37°C | - | 5 | 5 |

The examples referred to above were prepared in the following manner:-
1. De-ionised water was placed in a suitable vessel.
2. The chelating agent was dissolved in the water
3. The primary surfactant and foam booster were slowly added to the solution with stirring. Stirring was continued until all materials were completely dissolved.
4. The colorant was added to the mixture until completely dispersed.
5. The preservative and glycerin were added to the mixture and stirred until completely dissolved
6. The skin conditioning agent and Fragrance were premixed until homogeneous
7. The premix was then added to the mixture and stirred until completely dissolved.
8. The non-ionic gelling agent was added to the mixture and stirred until fully dissolved.
9. The pH was adjusted to pH 5.0 - 6.0 with Citric acid.
10. The post foaming agent was stirred into the mixture.
11. The mixture was immediately filled into a suitable aerosol can for a post foaming gel composition.
12. The gel was dispensed from the can at regular intervals following filling and graded for gel rigidity. The time taken to achieve a maximum gel rigidity was recorded.
It is of course to be understood that the invention is not intended to be restricted to the details of the above embodiments which are described by way of example only.

## Claims

1. A method for the manufacture of a post-foaming cleansing composition comprising the steps of:- adding at least one non-ionic gelling agent to a mixture comprising at least one anionic surfactant such that the ratio of anionic surfactants to non-ionic gelling agent is 4:1 or greater, the method further comprising combining the ensuing mixture with at least one post-foaming agent and filling the mixture into a package prior to the formation of a gel structure, and wherein a gel structure is formed at least 4 minutes after the addition of the post-foaming agent to the mixture, said gel structure being retained for at least 12 months following manufacture when the composition is stored at about 20°C to 25°C or below.

2. A method according to claim 1, wherein the gel rigidity of the composition remains substantially unchanged for at least 10 minutes after addition of the post-foaming agent to the remainder of the composition.

3. A method for the manufacture of a post-foaming cleansing composition according to claim 1 or claim 2, wherein the non-ionic gelling agent is selected from any oaf the following either alone or in combination:- laureth-2, laureth-4, C12/13 pareth-3, ceteareth-4 or oleth-3 or one or more glycol esters.

4. A method for the manufacture of a post-foaming cleansing composition as claimed in any preceding claim, wherein the non-ionic gelling agent consists of laureth-4.

5. A method for the manufacture of a post-foaming cleansing composition according to any preceding claim, wherein the non-ionic gelling agent constitutes from 0.01 % to 8.0% by weight of the total composition.

6. A method for the manufacture of a post-foaming cleansing composition according to any preceding claim, wherein the total amount of surfactant constitutes from 0.01% to 30.0% by weight of the total composition.

7. A method for the manufacture of a post-foaming cleansing composition according to any-preceding claim, wherein the post-foaming agent comprises at least one saturated aliphatic hydrocarbon having from 4 to 6 carbons.

8. A method for the manufacture of a post-foaming cleansing composition according to any preceding claim, wherein the post-foaming agent constitutes from 0.01% to 14% by weight of the total composition.

## Patentansprüche

1. Verfahren zur Herstellung einer nachschäumenden Reinigungsmittelzusammensetzung, umfassend die Schritte, dass zumindest ein nichtionisches Geliermittel zu einem Gemisch zugegeben wird, welches zumindest einen anionischen oberflächenaktiven Stoff umfasst, so dass das Verhältnis von anionischen oberflächenaktiven Stoffen zu nichtionischem Geliermittel 4:1 oder mehr beträgt, wobei das Verfahren des Weiteren umfasst, dass das entstehende Gemisch mit zumindest einem nachschäumenden Mittel kombiniert und das Gemisch vor der Bildung einer Gelstruktur in eine Verpackung gefüllt wird, und wobei eine Gelstruktur zumindest 4 Minuten nach der Zugabe des nachschäumenden Mittels zu dem Gemisch gebildet wird, wobei die Gelstruktur für mindestens 12 Monate nach der Herstellung erhalten bleibt, wenn die Zusammensetzung bei etwa 20°C bis 25°C oder darunter gelagert wird.

2. Verfahren nach Anspruch 1, wobei die Gelfestigkeit der Zusammensetzung nach Zugabe des nachschäumenden Mittels zu der übrigen Zusammensetzung für zumindest 10 Minuten im Wesentlichen unverändert bleibt.

3. Verfahren zur Herstellung einer nachschäumenden Reinigungsmittelzusammensetzung nach Anspruch 1 oder Anspruch 2, wobei das nichtionische Geliermittel aus einem der Folgenden allein oder in Kombination ausgewählt ist: Laureth-2, Laureth-4, C12/13 Pareth-3, Ceteareth-4 oder Oleth-3 oder ein oder mehrere Glykolester.

4. Verfahren zur Herstellung einer nachschäumenden Reinigungsmittelzusammensetzung nach einem der vorhergehenden Ansprüche, wobei das nichtionische Geliermittel aus Laureth-4 besteht.

5. Verfahren zur Herstellung einer nachschäumenden Reinigungsmittelzusammensetzung nach einem der vorhergehenden Ansprüche, wobei das nichtionische Geliermittel von 0,01 Gew.-% bis 8,0 Gew.-% der gesamten Zusammensetzung ausmacht.

6. Verfahren zur Herstellung einer nachschäumenden Reinigungsmittelzusammensetzung nach einem der vorhergehenden Ansprüche, wobei die gesamte Menge an oberflächenaktiven Stoffen von 0,01 Gew.-% bis 30,0 Gew.-% der gesamten Zusammensetzung ausmacht.

7. Verfahren zur Herstellung einer nachschäumenden Reinigungsmittelzusammensetzung nach einem der vorhergehenden Ansprüche, wobei das nachschäumende Mittel zumindest einen gesättigten aliphatischen Kohlenwasserstoff mit 4 bis 6 Kohlenstoffatomen umfasst.

8. Verfahren zur Herstellung einer nachschäumenden Reinigungsmittelzusammensetzung nach einem der vorhergehenden Ansprüche, wobei das nachschäumende Mittel von 0,01 Gew.-% bis 14 Gew.-% der gesamten Zusammensetzung ausmacht.

## Revendications

1. Procédé pour la production d'une composition nettoyante à pouvoir moussant différé, comprenant les étapes d'addition d'au moins un agent gélifiant non ionique à un mélange comprenant au moins un tensioactif anionique, de telle façon que le rapport entre tensioactifs anioniques et agent gélifiant non ionique soit de 4 : 1 ou plus, le procédé comprenant, en outre, l'association du mélange résultant avec au moins un agent à pouvoir moussant différé, l'introduction du mélange dans un emballage avant la formation d'une structure gélifiée, et dans lequel une structure gélifiée est formée au moins 4 minutes après l'addition de l'agent à pouvoir moussant différé au mélange, ladite structure gélifiée étant maintenue pendant au moins 12 mois après la production, lorsque la composition est conservée à environ 20 °C à 25 °C ou moins.

2. Procédé selon la revendication 1, dans lequel la rigidité du gel de la composition reste à peu près inchangée pendant au moins 10 minutes après l'addition de l'agent à pouvoir moussant différé au reste de la composition.

3. Procédé pour la production d'une composition nettoyante à pouvoir moussant différé selon la revendication 1 ou la revendication 2, dans lequel l'agent gélifiant non ionique est choisi parmi les composés suivants , seuls ou en association : le laureth-2, le laureth-4, un pareth-3 en C12/13, le ceteareth-4 ou l'oleth-3, ou un ou plusieurs esters glycoliques.

4. Procédé pour la production d'une composition nettoyante à pouvoir moussant différé selon l'une quelconque des revendications précédentes, dans lequel l'agent gélifiant non ionique consiste en laureth-4.

5. Procédé pour la production d'une composition nettoyante à pouvoir moussant différé selon l'une quelconque des revendications précédentes, dans lequel l'agent gélifiant non ionique représente de 0,01 à 8,0 % en poids de la composition totale.

6. Procédé pour la production d'une composition nettoyante à pouvoir moussant différé selon l'une quelconque des revendications précédentes, dans lequel la quantité totale de tensioactif représente de 0,01 à 30,0 % en poids de la composition totale.

7. Procédé pour la production d'une composition nettoyante à pouvoir moussant différé selon l'une quelconque des revendications précédentes, dans lequel l'agent à pouvoir moussant différé comprend au moins un hydrocarbure aliphatique saturé comportant de 4 à 6 atomes de carbone.

8. Procédé pour la production d'une composition nettoyante à pouvoir moussant différé selon l'une quelconque des revendications précédentes, dans lequel l'agent à pouvoir moussant différé représente de 0,01 % à 14 % en poids de la composition totale.
